# EUROPEAN PATENT APPLICATION

(11) **EP 1 201 260 A1**
(43) Date of publication of application: **02.05.2002**
(21) Application number: 01402698.3
(22) Date of filing: 18.10.2001
(51) Int. Cl.: A61M 25/01

(54) **Rapid exchange delivery catheter**

(30) Priority: 18.10.2000 US 241501 P; 17.10.2001 US 981769
(71) Applicant: Oslund, John C., Cottage Grove, Minnesota 55016 (US); Bowden, Russel W., Tyngsboro, Massachusetts 0189 (US)
(72) Inventor: Oslund, John C., Cottage Grove, Minnesota 55016 (US); Bowden, Russel W., Tyngsboro, Massachusetts 0189 (US)
(74) Representative: Geismar, Thierry

(57) **Abstract**

The present invention relates generally to the field of delivery catheter devices, procedures and interventions. In particular, the present invention provides a delivery catheter and a method for using a delivery catheter to deliver a distal protection device across a stenosis through use of a rapid exchange length guidewire. The delivery catheter comprises a catheter body having a first lumen receiving a guidewire reciprocating in the first lumen, a second lumen receiving a distal protection device reciprocating in the second lumen, and distal lumen connected to and in communication with the first lumen and second lumen.

## Description

### CROSS-REFERENCE TO RELATED APPLICATIONS

This is a regular application filed under 35 U.S.C. § 111(a) claiming priority, under 35 U.S.C. § 119 (e) (1), of provisional application Serial No. 60/241,501, previously filed October 18, 2000, under 35 U.S.C. § 111(b).

### FIELD OF THE INVENTION

The present invention relates generally to the field of delivery catheter devices, procedures and interventions. In particular, the present invention provides a delivery catheter and a method for using a delivery catheter to deliver a distal protection device across a stenosis through use of a guidewire.

### BACKGROUND OF THE INVENTION

A substantial health risk exists when deposits of fatty-like substances, referred to as atheroma or plaque, accumulate on the wall of a blood vessel. A stenosis is formed where such deposits restrict or occlude the flow of blood through the blood vessel. Various therapeutic devices may be deployed to treat a stenosis including balloon angioplasty catheters, stents, drug coated stents, atherectomy catheters, laser ablation catheters, drug delivery catheters, and the like. In some of these procedures, there is a risk that a deposit may dislodge causing particulate matter to become entrained in the blood stream. Once entrained, the particulate matter may travel downstream and cause a blockage or restrict flow to a smaller blood vessel elsewhere in the vascular system possibly causing a stroke or heart attack. This risk can be prevented by placing a distal embolic protection device downstream of the stenosis prior to the deployment of a therapeutic device to treat the stenosis.

A protection device generally has a host guidewire comprising an elongate shaft, and a working member located at a distal region of the host guidewire. The working member comprises an expandable and collapsible filter or screen having a plurality of pores. In an expanded configuration, the working area expands outwardly from the host guidewire to form a screen or filter having a plurality of pores. The expanded screen has a diameter at least as large as that of the blood vessel such that it engages the wall of the blood vessel and traps and prevents passage of particulate matter while allowing passage of a fluid such as a patient's blood. The screen may take a variety of shapes such as a windsock, a basket, a basket with a lid, several shapes in series or other such suitable configurations known in the art. The screen has a collapsed configuration wherein the screen, having a periphery, is collapsed towards the host guidewire. The collapsed configuration has a smaller diameter thus allowing the distal protection device to be loaded into a delivery catheter and advanced into the blood vessel of a patient's body. The collapsed configuration has a smaller diameter yet it is still significantly larger than the diameter of a guidewire. Thus, crossing a stenosed region is more difficult with a distal protection device than with a guidewire. For this reason, it is preferable to first cross a stenosis with a guidewire to gain access to a position downstream of a stenosis.

Traditional methods for gaining access downstream of a stenosis to allow the placement of a distal protection device typically require several steps. First, access to a blood vessel or artery is generally obtained by a puncture site in a blood vessel such as the femoral artery located in a patient's inner thigh. A guide catheter, typically 100 cm long, and a guidewire, typically 145-320 cm long, are then introduced into a patient's vascular system at the puncture site. The guide catheter and guidewire are then advanced into the vascular system to a position proximal to a stenosis, thus creating access to the stenosis for a vascular device, such as a catheter, for treatment or diagnosis.

Many vascular devices require access distal to a stenosis. In most cases, access distal to the stenosis is obtained by advancing the guidewire across the stenosis and then feeding the vascular device over the guidewire until the device is positioned distal to the stenosis. In this sense, a device is positioned "distal" to the stenosis when the device has a distal region that is positioned "distal" to the stenosis with respect to the puncture site, wherein the puncture site is proximal to the stenosis. The crossing of the stenosis with a guidewire is preferred because a guidewire has a lower crossing profile than the delivery catheter carrying the working device.

A preferred method for positioning a protection device includes use of a guidewire to cross a stenosed region. Once the stenosis is crossed with the guidewire, the guidewire is removed and a delivery catheter having a pre-loaded distal protection device is delivered into the blood vessel and across the stenosis. The distal protection device is then deployed distal to the stenosis. Once the distal protection device has been deployed, the delivery catheter is completely removed from the blood vessel.

Three different methods are commonly employed for removal of the delivery catheter. The first method is termed an "over the wire" method. This method requires that a host guidewire of the distal protection device have a length, for example, greater than 3 meters, with as much as 1.5 meters of which being advanced into a patient's vascular system. With a requirement that the guidewire be more than twice the catheter length, a delivery catheter having a length of greater than 1.5 meters so as to allow an operator to position the distal protection device at the stenosis will require the host guidewire to have a length of greater than 3 meters. As a result, when the delivery catheter is removed, the operator is able to maintain the control and position of the host guidewire relative to the delivery catheter.

A second commonly utilized method for removing the delivery catheter is a "rapid exchange" method. This method typically has a 21 cm rapid exchange lumen in the distal portion of the catheter only.

In a third method, the delivery catheter is able to be peeled along its length. For example, for a 1.5 meter host guidewire having 0.5 meters extending outside the patient's body and therefore accessible to the operator, the delivery catheter can be removed by peeling away the catheter, thus allowing the operator to maintain control of host guidewire relative to the delivery catheter with a shorter host guidewire length than with the "over the wire" method.

The greatest disadvantage of these devices and methods is that the guidewire must be removed prior to insertion of a distal protection device. Additionally, the "over the wire" method requires a long host guidewire making removal of the delivery catheter more difficult, whereas the "rapid exchange" delivery catheters that peel away upon removal tend to lack axial strength.

### SUMMARY OF THE INVENTION

The present invention provides a "rapid exchange" style delivery catheter for crossing a stenosed region using a guidewire for placement of a distal protection device. A distal protection device, suitable for use with the delivery catheter and method of the present invention, is disclosed in Mazzocchi, PCT Int'l Pub. No. WO 96/01591, Int'l Pub. Date Jan. 25, 1996, assigned to Microvena, Corp., and its description is hereby incorporated by reference. The delivery catheter has a catheter body that includes a distal lumen connected to and in communication with a proximal dual-lumen section. The dual-lumen section includes a first lumen, being a guidewire lumen, adjacent a second lumen, being a device lumen. The first lumen has a distal end and the second lumen has a distal end adjacent that of the first lumen. The first lumen has a length greater than that of the second lumen such that the first lumen extends proximal to the second lumen.

The distal lumen has a diameter that increases as it progresses from a distal tip allowing it to connect to and communicate with the first lumen and the second lumen. The dual-lumen structure allows for the receiving of a guidewire wherein the guidewire reciprocates within the first lumen. The dual-lumen structure also allows for the receiving of a distal protection device wherein the distal protection device reciprocates within the second lumen. The guidewire may be advanced through the first lumen to a position distal to the distal tip of the catheter. Likewise, the distal protection device may be advanced through the second lumen to a position distal to the distal tip.

In use, the guidewire is pre-loaded into the first lumen and the distal protection device is pre-loaded into the second lumen. The guidewire and distal protection device are advanced to the distal ends of the first lumen and second lumen, respectively. The delivery catheter is then advanced into a lumen, such as a blood vessel of a patient's body, at a position upstream of the stenosis, to a position proximal to the stenosis. Once positioned, with the delivery catheter remaining stationary, the guidewire is advanced through the distal tip of the distal lumen, and across the stenosed region, to a position downstream of and distal to the stenosis. The guidewire is then held stationary in this position while the delivery catheter is advanced over the guidewire to a position distal to the stenosis with respect to the catheter entry location. The guidewire has thus been used to cross the stenosis and position the delivery catheter distal to the stenosis. The guidewire is then retracted into the guidewire lumen while the distal tip of the delivery catheter remains stationary, distal to the stenosis. The delivery catheter is now in position to deliver the distal protection device. The distal protection device is advanced from the delivery catheter, into the patient's lumen, to a position distal to the stenosis. The distal protection device is then deployed distal to the stenosis.

The distal protection device has a small diameter configuration during delivery and a larger expanded configuration upon deployment. Upon deployment the distal protection device has a peripheral diameter that expands outwardly producing a larger diameter. The lumen of a patient's body has a wall with a diameter. The diameter of the distal protection device is at least as large as the lumen diameter wherein the diameter of the distal protection device contacts the lumen wall. This secures the distal protection device in the lumen so as to prevent the passage of emboli and particulate matter distal to the stenosed region. In this expanded configuration the distal protection device acts as a filter having a plurality of pores for the passage of fluid, such as blood, through the lumen while preventing passage of particulate matter that is larger than the pores in the filter.

The delivery catheter is then removed from the patient's lumen using a "rapid exchange" method of removal. The delivery catheter is removed by sliding the delivery catheter in the proximal direction, over the length of the host guidewire of the distal protection device. A proximal portion of the host guidewire protrudes externally from a patient's body. The second lumen, sliding proximally over the host guidewire, has a length shorter than the first lumen. Thus, the proximal portion of the host guidewire need only be minimally longer than the second lumen and the distal lumen in order to remove the delivery catheter from the host guidewire. The distal protection device is secured distal to the stenosed region and the host guidewire can then be used for access to the stenosed region for treatment or diagnosis with any suitable treatment or diagnostic devices known in the art.

### BRIEF DESCRIPTION OF THE DRAWINGS

FIG. 1 illustrates an embodiment of the present invention wherein a delivery catheter has two tubular bodies connected to and in communication with a distal tubular body forming a "rapid exchange" style catheter with a pre-loaded guidewire in a first lumen and a pre-loaded distal protection device in a second lumen;
FIG. 2 illustrates another embodiment of the delivery catheter of the present invention having a single tubular member with a first lumen and a second lumen; and
FIG. 3 illustrates another embodiment of the delivery catheter of the present invention having a first tubular member and a flared tubular member receiving a portion of the first tubular member.

### DETAILED DESCRIPTION OF THE PREFERRED EMBODIMENTS

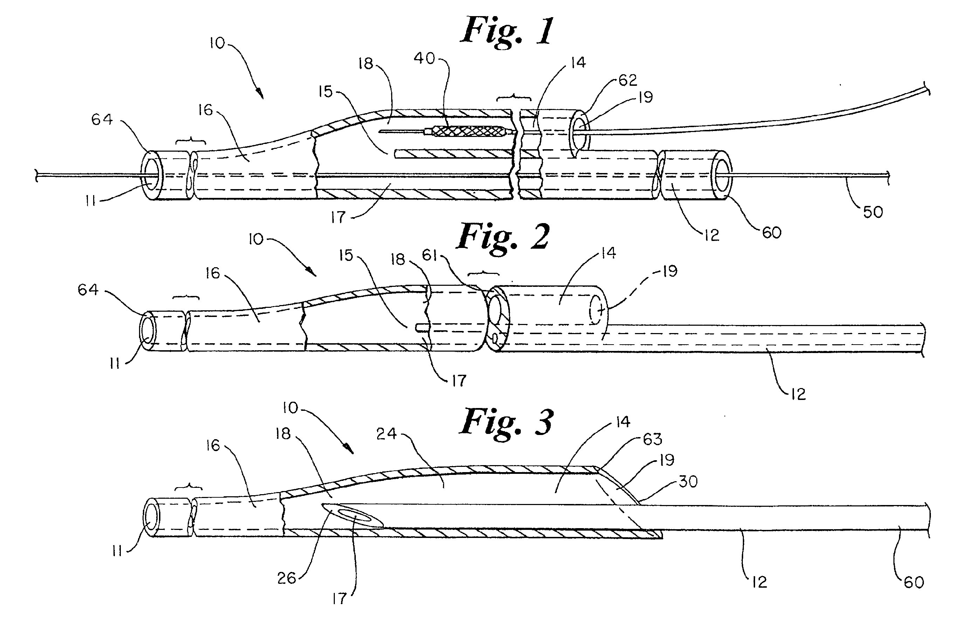

FIG. 1 illustrates a delivery catheter 10 having a catheter body comprising a first tubular member 60 defining a first lumen 12, a second tubular member 62 defining a second lumen 14, and a distal tubular member 64 defining a distal lumen 16. The first tubular member 60 and first lumen 12 have a distal end 17 and a proximal end (not shown). The second tubular member 62 and second lumen 14 have a distal end 18 and a proximal end 19. First lumen 12 and second lumen 14 are adjacent one another and have distal ends 17 and 18 adjacent one another. First lumen 12 extends proximally with respect to lumen 14, where first lumen 12 has a length and second lumen 14 has a lesser length. The distal tubular member 64 and distal lumen 16 have a proximal end 15 and a distal end or distal tip 11, wherein the proximal end 15 is connected to, and in communication with, the first lumen 12 and second lumen 14. The distal lumen 16 has a diameter that is shown as increasing as it progresses proximally to encompass the first lumen distal end 17 and second lumen distal end 18.

The first lumen 12 has a predetermined length. The second lumen 14 has a length less than the predetermined length of first lumen 12. First lumen 12 runs adjacent to second lumen 14 and terminates such that first lumen distal end 17 is adjacent second lumen distal end 18. Distal lumen 16 is connected to, and in communication with, the distal ends of first lumen 17 and second lumen 18. Distal lumen 16 has a diameter at proximal end 15 and a lesser diameter at distal end 11. In one embodiment, the diameter decreases along the length of distal lumen 16 in a distal direction. In another embodiment, the diameter decreases along the length of distal lumen 16 in the distal direction until a predetermined diameter at which the diameter is maintained along the remaining length of distal lumen 16 in the distal direction. In the embodiment shown in FIG. 1, a portion of distal lumen 16 is aligned with a portion of first lumen 12 and wherein a portion of distal lumen 16 in communication with second lumen 14 tapers along at least a portion of the length of distal lumen 14 in a distal direction so as to decrease diameter of distal lumen 16 as it progresses in the distal direction.

The first lumen 12 may have a tapered luer fitting at the proximal end (not shown) for receiving a device such as a guidewire 50 shown in FIG. 1. The guidewire 50 has a distal end front-loaded into first lumen 12 such that the distal end of guidewire 50 is adjacent first lumen distal end 17. The guidewire 50 reciprocates within first lumen 12 such that guidewire 50 may advance into distal lumen 16 and advance distal to distal tip 11. The guidewire 50 may retract into first lumen 12 and may be fully removed from the first lumen proximal end (not shown).

The second lumen 14 has a proximal end 19 for receiving a working device, such as a distal protection device 40 having a distal end, as shown in FIG. 1. The distal protection device 40 is loaded into second lumen 14 such that the distal end of the distal protection device is located near the second lumen distal end 18. The distal protection device 40 can be positioned anywhere in lumen 18 during delivery of the device. The distal protection device 40 reciprocates within second lumen 14 such that distal protection device 40 may advance into distal lumen 16 and advance distal to distal tip 11. The delivery catheter 10 and guidewire 50 may be withdrawn over the length of the distal protection device 40 in the proximal direction thus leaving distal protection device 40 in place distal to delivery catheter 10,

The delivery catheter 10 in FIG. 1 may be used in a vessel of a human body such as a blood vessel for treatment of a stenosis. The delivery catheter 10 has a pre-loaded guidewire 50 in first lumen 12 and a pre-loaded distal protection device 40 in second lumen 14, wherein the distal end of distal protection device 40 is adjacent to the distal end of the guidewire 50 and adjacent to the distal ends of first lumen 17 and second lumen 18. The pre-loaded delivery catheter 10 is advanced into a patient's blood vessel to a position proximal to the stenosis. The guidewire 50 is advanced through distal lumen 16 and distal to tip 11. The guidewire 50 then crosses the stenosis to a position distal to the stenosis. The delivery catheter 10 is then advanced over the guidewire 50 such that tip 11 is distal to the stenosis. The guidewire 50 is then retracted into the first lumen 12. The distal protection device 40 is then advanced distal to tip 11. The diatal protection 40 device is deployed in the blood vessel. The delivery catheter 10 is withdrawn over the length of the distal protection device 40 in the proximal direction thus removing delivery catheter 10 and guidewire 50 in a "rapid exchange" style from the blood vessel and leaving the distal protection device 40 in the blood vessel. The purpose of the delivery catheter 10 is not limited to use in treatment of a stenosis.

The first tubular member 60 may be attached to the second tubular member 62 using a heat shrink tubing encircling a length of the two tubular bodies. Such a method of securing said tubular members, 60 and 62, is known to those of ordinary skill in the art. The distal tubular member 64 may also be secured to the first and second tubular bodies, 60 and 62, by heat bond or adhesive bond, or other methods known to those of ordinary skill in the art. The delivery catheter components can be made of any suitable materials known in the art. Materials having low surface friction are preferable, such as HDPT or those lined with PTFE, Braid reinforcement may also be utilized over all or part of the length of the delivery catheter 10.

FIG. 2 illustrates another embodiment of the current invention similar to the embodiment of FIG. 1. The delivery catheter 10 in FIG. 2 has a catheter body having a dual-lumen tubular member 61 having a first lumen 12 of a length, and a second lumen 14 having a length less than that of first lumen 12. The first lumen 12 has a distal end 17 and a proximal end (not shown). The second lumen 14 has a distal end 18 and a proximal end 19. The first lumen 12 runs adjacent to the second lumen 14 and the distal end of the first lumen 17 is adjacent to the distal end of the second lumen 18. The first lumen proximal end (not shown) is proximal to the second lumen proximal end 19.

The catheter body has a distal tubular member 64 having a distal lumen 16 that is connected to and in communication with first lumen 12 and second lumen 14. The distal lumen 16 is a single lumen extending from a proximal end 15 having a diameter that decreases as it progresses towards a distal end 11, or distal tip 11, having a lesser diameter. The diameter may decrease over the entire length of the distal lumen 16 or only a portion of the length of the distal lumen.

The first lumen 12 may have a tapered luer fitting at the proximal end (not shown) for receiving a device such as a guidewire 50 shown in FIG. 1. The guidewire 50 has a distal region that is front-loaded into first lumen 12 such that the distal region of guidewire 50 is adjacent first lumen distal end 17. Guidewire 50 reciprocates within first lumen 12 such that the distal region of guidewire 50 may advance into distal lumen 16 and advance distal to distal tip 11. Guidewire 50 may then retract into first lumen 12 and may be fully removed from the first lumen proximal end (not shown).

The second lumen 14 has a proximal end 19 for receiving a working device such as a distal protection device 40. The distal protection device 40 has a distal region with a working area as shown in FIG. 1. The distal protection device 40 is front-loaded into second lumen 14 such that the distal region of the distal protection 40 device is adjacent second lumen distal end 18. The distal protection device 40 reciprocates within second lumen 14 such that the distal region of distal protection device 40 may advance into distal lumen 16 and advance distal to distal tip 11. Once advanced, distal protection device 40 may be deployed distal to distal tip 11. The delivery catheter 10 and guidewire 50 may then be withdrawn over the length of the distal protection device 40 by sliding delivery catheter 10 over distal protection device 40 in the proximal direction.

The delivery catheter 10 in FIG. 1 may be used in a lumen of a human body such as a blood vessel for use in treatment of a stenosis. The delivery catheter 10 has a pre-loaded guidewire 50 in first lumen 12 and a pre-loaded distal protection device 40 in second lumen 14 wherein the distal region of the distal protection device 40 is adjacent to the distal region of guidewire 50 and adjacent to. the distal ends of first lumen 17 and second lumen 18. The pre-loaded delivery catheter 10 is advanced in the blood vessel to a position proximal to the stenosis. The distal region of the guidewire 50 is advanced through distal lumen 16 and distal to distal tip 11. The guidewire 50 crosses the stenosis to a position distal to the stenosis. The guidewire 50 is then held stationary while the delivery catheter 10 is advanced over the guidewire 50 such that distal tip 11 is distal to the stenosis. The guidewire 50 is then retracted into first lumen 12. The distal protection device is then advanced distal to distal tip 11. The distal protection device 40 is deployed in the blood vessel. The delivery catheter 10 is then withdrawn by sliding the delivery catheter 10 in the proximal direction over the length of distal protection device 40 thus removing delivery catheter 10 and guidewire 50 in a "rapid exchange" style from the blood vessel and leaving distal protection device 40 deployed in the blood vessel. The use of the delivery catheter 10 in treatment of a stenosis merely illustrates a use of the present invention and does not limit the invention to such use.

Construction of a dual-lumen tubular member 61 as depicted in FIG. 2 is common to those of ordinary skill in the art. A method of forming a single lumen section and a dual-lumen section from a dual-lumen tubular member 61 is common to those of ordinary skill in the art. The distal tubular member 64 is secured to the dual-lumen tubular member 61 by heat bond, RF, adhesive bond or other methods of which are common to those of ordinary skill in the art. The delivery catheter components can be made of any of various materials common in the art. Materials having low surface friction are preferable such as those lined with PTFE. Also, it is possible to have a braided reinforcement over all or part of the length of the delivery catheter 10.

FIG. 3 illustrates yet another embodiment of the present invention. FIG. 3 illustrates a catheter body comprising a flared tubular member 63, having a flared lumen 24, receiving a portion of a first tubular member 60 having a first lumen 12, thus forming a delivery catheter 10 having a proximal section, a dual-lumen section, and a distal section.

The flared tubular member 63 has a flared lumen 24 having a proximal end 30 and a distal end 11 and a length and a cross-sectional area that decreases at it progresses towards the distal end 11. The flared tubular member 63 receives a portion of first tubular member 60 at proximal end 30. The first tubular member 60 has a first lumen 12 and a diameter less than the cross-sectional area of the flared lumen 24. The first tubular member 60 and first lumen 12 extend proximal to flared lumen proximal end 30 forming a proximal section. The portion of first tubular member 60 that is received by the flared lumen 63 forms a dual-lumen section comprising the first lumen 12 and a second lumen 14. The second lumen 14 comprises the cross-sectional area of the flared lumen 24 adjacent first tubular member 60. The second lumen 14 is adjacent the first lumen 12 and has a proximal end 19 and a distal end 18 adjacent first lumen distal end 17, A portion of flared lumen 24 extends distal to the dual-lumen section and forms a distal lumen 16 having a distal tip 11.

The flared tubular member 63 shown in FIG. 3 has a proximal end 30 such that the portion in contact and secured to the first tubular member 60 has a greater length than the portion not in contact with the first tubular member 60. The flared tubular member 60 has a cross-sectional area that decreases as it progresses in the distal direction. In an alternative embodiment, the cross-sectional area beginning at flared lumen proximal end 30 may remain constant for a given length in the distal direction, taper for a given length, and then remain at a constant smaller cross-sectional area extending to distal tip 11. Other variations of decreasing the cross-sectional area of the flared lumen 24 in the distal direction may be used and are not limited to those disclosed herein.

The first lumen 12 may have a tapered luer fitting at the proximal end (not shown) for receiving of a device such as a guidewire 50 shown in FIG. 1. The guidewire 50 has a distal region that is front-loaded into first lumen 12 such that the distal region of guidewire 50 is adjacent first lumen distal end 17. The guidewire 50 reciprocates within first lumen 12 such that the distal region of guidewire 50 may advance into distal lumen 16 and advance distal to distal tip 11. Guidewire 50 may then retract into first lumen 12 and may be fully removed from the first lumen proximal end (not shown).

The second lumen 14 has a proximal end 19 for receiving a working device such as a distal protection device 40 that has a distal region with a working area, as shown in FIG. 1. The distal protection device 40 is front-loaded into second lumen 14 such that the distal region of distal protection device 40 is adjacent second lumen distal end 18. The distal protection device 40 reciprocates within second lumen 14 such that the distal region of distal protection device 40 may advance into distal lumen 16 and advance distal to distal tip 11. Once advanced, distal protection device 40 may be deployed distal to distal tip 11. The delivery catheter 10 and guidewire 50 may then be withdrawn over the length of the distal protection device 40 by sliding delivery catheter 10 over distal protection device 50 in the proximal direction. The first lumen distal end 17 may have a bevel 26 such that the portion of the first tubular member 60, in contact with flared tubular member 63, has a length less than that of the portion not in contact, thus forming bevel 26. The distal lumen 16 is located distal to bevel 26. The bevel 26 can be used to create a positive stop for distal protection device 40 wherein the distal protection device 40 cannot advance into distal lumen 16 without deforming bevel 26. The bevel 26 could also be beneficial for initially back-loading a guidewire, since it would prevent the guidewire from entering the protection device lumen during initial back-loading. The bevel 26 is a flexible or resilient material that allows distal protection device 40 to advance into distal lumen 16 only upon deforming or bending of bevel 26 The bevel 26 prevents the distal protection device 40 from advancing distal to bevel 26 until a deforming force is applied to the distal protection device 40 sufficient to deform bevel 26.

The delivery catheter 10 illustrated in FIG. 3 may be used in a lumen of a human body such as a blood vessel for use in treatment of a stenosis. The delivery catheter 10 has a pre-loaded guidewire 50 in first lumen 12 and a pre-loaded distal protection device 40 in second lumen 14 wherein the distal region of distal protection device 40 is adjacent to the distal region of guidewire 50 and adjacent distal end of first lumen 17 and second lumen 18. The pre-loaded delivery catheter 10 is advanced into the blood vessel to a position proximal to the stenosis. The guidewire 50 distal region is advanced through distal lumen 16 and distal to distal tip 11. The guidewire 50 crosses the stenosis to a position distal to the stenosis. The delivery catheter 10 is then advanced over the guidewire 50 such that distal tip 11 is distal to the stenosis. The guidewire 50 is then retracted into first lumen 12. The distal protection device 40 is then advanced distal to distal tip 11 by advancing across bevel 26 by deforming bevel 26. The distal protection device 40 is deployed in the blood vessel. The delivery catheter 10 is withdrawn proximally over length of distal protection device 40 thus removing delivery catheter 10 and guidewire 50 in a "rapid exchange" style from the blood vessel and leaving distal protection device 40 deployed in the blood vessel. The use of the delivery catheter 10 in the treatment of the stenosis merely illustrates a use of the delivery catheter 10 and does not limit such use to treatment of a stenosis.

The first tubular member 60 is secured to the flared tubular member 63 using an adhesive type bond or a heat bond or any suitable method of securing the tubular members known to those of ordinary skill in the art. The delivery catheter components can be made of any of suitable materials known in the art. For example, materials having low surface friction are preferable such as those lined with PTFE, Additionally, braid reinforcement may be utilized over all or part of the length of the delivery catheter 10.

It will be understood that this disclosure, in many respects, is only illustrative. Changes may be made in details, particularly in matters of shape, size, material, and arrangement of parts without exceeding the scope of the invention. Accordingly, the scope of the invention is as defined in the language of the appended claims.

## Claims

1. A catheter for delivery of a medical device, comprising:
a catheter body defining:
a first lumen having a proximal end, a distal end, and a length;
a second lumen adjacent the first lumen, said second lumen having a distal end adjacent said first lumen distal end and a length less than that of said first lumen length; and
a distal lumen connected to and in communication with said first lumen and said second lumen.

2. The delivery catheter in accordance with Claim 1 further comprising a guidewire pre-loaded in said first lumen of said catheter body, and a medical device pre-loaded in said second lumen of said catheter body.

3. The delivery catheter according to claim 2 wherein, said guidewire passes through said first lumen and said distal protection device passes through said second lumen.

4. The delivery catheter according to claim 2 wherein, said distal lumen has a cross-sectional area which decreases as it progresses in distal direction.

5. The delivery catheter according to claim 2 wherein *said* first lumen is defined within a tubular member having a distal end, and wherein said tubular member distal end is beveled to define an oblique surface.

6. The delivery catheter of claim 5 wherein said beveled surface allows back-loading of said guidewire.

7. The delivery catheter according to claim 5 wherein said medical device, when advanced into said distal lumen, deforms said oblique surface.

8. The delivery catheter according to claim 2 wherein said guidewire and said medical device have a rapid exchange style configuration.

9. The delivery catheter according to claim 2 wherein said first lumen is located in a first tubular body, said second lumen is located in a second tubular body and said distal lumen is located in a distal tubular body.

10. , The delivery catheter according to claim 2, wherein said first lumen and said second lumen are located in a dual-lumen tubular body, and said distal lumen is located in a distal tubular body.

11. The delivery catheter according to claim 2, wherein said first lumen is located in a first tubular body and said second lumen and said distal lumen are located in a flared tubular body.

12. A delivery catheter for use in a blood vessel of a patient's body for delivery of a medical device across a stenosed region, comprising;
a catheter body having a first lumen receiving a guidewire, said guidewire movable along said first lumen, said guidewire is advanced into a distal tubular body having a distal lumen in contact and communication with said first lumen, wherein said distal lumen being proximal to a stenosis, wherein said guidewire advances distal to the stenosis and said delivery catheter is advanced over said guidewire to a location distal to the stenosis, said guidewire being retractable into said first lumen;
said catheter body having a second lumen in communication with said distal lumen, said second lumen receiving a distal protection device, said distal protection device being movable in said second lumen, wherein said distal protection device is advanceable through said distal lumen to a location distal to the stenosis, wherein said delivery catheter is retractable over said distal protection device in a proximal direction such that said delivery catheter and said guidewire are removable from the blood vessel.
